(19) Europäisches Patentamt
European Patent Office
Office européen des brevets

(11) **EP 4 042 402 B1**

(12) **EUROPEAN PATENT SPECIFICATION**

(45) Date of publication and mention
of the grant of the patent:
**29.11.2023 Bulletin 2023/48**

(21) Application number: **20797533.5**

(22) Date of filing: **05.10.2020**

(51) International Patent Classification (IPC):
**G09B 23/28** (2006.01)    **A61B 34/20** (2016.01)
**A61B 34/10** (2016.01)    **G09B 23/30** (2006.01)
**A61B 90/00** (2016.01)    **A61B 90/50** (2016.01)

(52) Cooperative Patent Classification (CPC):
**G09B 23/285;** A61B 2090/365; A61B 2090/372;
A61B 2090/502; G09B 23/30

(86) International application number:
**PCT/FI2020/050655**

(87) International publication number:
**WO 2021/069795 (15.04.2021 Gazette 2021/15)**

(54) **APPARATUS AND METHOD FOR SURGERY PREPARATION**

GERÄT UND VERFAHREN ZUR OPERATIONSVORBEREITUNG

APPAREIL ET PROCÉDÉ DE PRÉPARATION À UNE CHIRURGIE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **07.10.2019  FI 20195854**

(43) Date of publication of application:
**17.08.2022  Bulletin 2022/33**

(73) Proprietor: **Osgenic Oy
00180 Helsinki (FI)**

(72) Inventor: **VARTIA, Olli
02760 Espoo (FI)**

(74) Representative: **Papula Oy
P.O. Box 981
00101 Helsinki (FI)**

(56) References cited:
**WO-A1-2016/109575    US-A1- 2010 178 644
US-A1- 2013 187 930**

**Description**

**FIELD**

The present invention relates to creating an environment for surgery preparation, including teaching and training of surgery.

**BACKGROUND**

Typically, surgery has been learned from text books and training with different types of samples, such as dead bodies. Lately, the development of computer technology has made it possible to create computerized training environments for various different fields. This includes also surgery, where a computerized training environment can be set up to prepare doctors or doctors-to-be for surgical procedures. However, the field of surgery differs from many others in that it typically has very high requirements for precision of the operation and the consequences of even a small failure may be severe. Moreover, the field deals with living tissue, which requires specific attention to be replicated in a computerized environment so that it can be effectively used for actual surgery preparation. Current methods for surgical cutting in a computerized environment are lacking in real-time operation. Document US 2010/178644 relates to the simulation of physical interactions with biological tissue in a similar surgical training system.

**OBJECTIVE**

An objective is to provide an improved computerized surgical environment.

In particular, it is to provide an improved computerized surgical environment an objective with a replication of tissue corresponding to a subject for surgery allowing more efficient surgery preparation.

In addition to general objectives, two detailed objectives are set for some embodiments. First, it is an objective to improve the replication of elasticity in the tissue to allow more efficient surgery preparation. Second, it is an objective to improve the replication of incompressibility in the tissue to allow more efficient surgery preparation. The improvements for elasticity and/or incompressibility can also provide a more realistic tissue for surgery preparation.

Finally, it is an objective to provide an improved computerized surgical environment allowing real-time replication of a surgical procedure.

**SUMMARY**

According to a first aspect, an apparatus for surgery preparation, including teaching of surgery and/or training for surgery, comprises a tracking device for representing a surgical instrument, such as a scalpel, and a display device for displaying a subject for surgery. The apparatus also comprises one or more processors arranged to receive signals corresponding to the tracking device to determine the position of a virtual surgical instrument following the movement of the tracking device. This allows a user, such as a doctor, to move the tracking device to guide the virtual surgical instrument and use it to perform a surgical operation in a computerized surgical environment. The apparatus can be arranged to use the display device to display the surgical operation in real-time.

The apparatus also comprises one or more memories comprising computer program code, wherein the one or more memories and the computer program code are configured to cause the one or more processors to perform the following in the indicated order and/or in any suitable order. First, generate a model for three-dimensional representation of a tissue corresponding to the subject for surgery, the model comprising plurality of tissue elements arranged to together represent the tissue. Second, determine a spatial location for each of the tissue elements with a local/global solver for a state equation of motion for the tissue elements, which local/global solver alternatingly obtains a set of local solutions under one or more constraints for the tissue elements indicating optimization, e.g. minimization, of an elastic potential for the tissue elements and determines the spatial locations which optimize the state equation when the elastic potential corresponds to the local solutions. Third, based on the position and, optionally, motion of the virtual surgical instrument with respect to the tissue elements, alter the one or more constraints to indicate that a cut has been made to the tissue with the virtual surgical instrument. These allow one or more surgical operations to be efficiently replicated substantially in real time as is described in more detail below in conjunction of the second aspect. In particular, the procedure allows a precise replication of various tissue types and their interactions with the virtual surgical instrument during a surgical operation, where the tissue is cut. Moreover, it allows a precise replication of the internal interactions of the tissue altering the shape of the tissue during the surgical operation.

According to a second aspect, a method is disclosed. The method can be used for surgery preparation, for example for teaching of surgery and/or training for surgery. The method comprises the following steps, which can be performed as follows in the indicated order and/or in any suitable order. One or more method steps can also be performed as part of another method step. The apparatus according to the first aspect can be arranged for performing the method, for example the one or more memories and the computer program code can be configured to cause the one or more processors to perform the method.

First, generate a model for three-dimensional representation of a tissue corresponding to a subject for surgery, the model comprising plurality of tissue elements arranged to together represent the tissue. This allows a replication of a tissue in a computerized surgical environment. The tissue may comprise one or more types of human tissue. In particular, it has been found that the method allows improved replication of soft tissue, such as fat tissue, skin tissue, muscle tissue and/or ligaments. While the representation allows discretization of the tissue into tissue elements, it is not necessarily tied to any particular representation, e.g. that of a polygonal mesh, such as a triangular mesh. As an example, the three-dimensional representation can comprise a mesh, such as a volumetric mesh, wherein a tissue element may correspond to a node. However, alternative or additionally, the three-dimensional representation may comprise a meshless structure of tissue elements. The tissue elements can thereby be formed as separate elements in three-dimensional space, each having a spatial location in three-dimensional space. There, interactions between tissue elements can correspond to one or more constraints, which may be soft constraints, i.e. they are not necessarily required to be fulfilled under all conditions. This allows the model to be particularly applicable for surgery, since a cut in the model does not require reformulation of a mesh, which may be particularly difficult due to cuts through three-dimensional elements. Instead, each of the tissue elements may correspond to a single spatial location allowing cuts to simply remove interactions between tissue elements without breaking the tissue elements. Using a three-dimensional representation with a meshless structure has been found particularly suitable for soft tissue, such as fat tissue. Generating the model should be understood to include the initial generation of the model and/or any later formation of the model from which information about the tissue elements can be obtained. Generating the model may include determining an initial state for the plurality of tissue elements before a cut or any cuts have been made to the tissue. The initial state may comprise the spatial locations of the tissue elements and/or any values derivable therefrom.

Second, determine a spatial location for each of the tissue elements with a local/global solver for a state equation of motion for the tissue elements. While the state equation of motion can be formed as corresponding to a force balance equation for the tissue elements, the local/global solver allows a solution for the state equation to be found without explicitly processing the full state equation. In particular, with the local/global solver the determination of forces affecting the tissue elements can be limited. The local/global solver also improves numerical solution of the state equation as it allows conservation of energy for the state equation even with extended time steps.

The local/global solver alternatingly obtains a set of local solutions under one or more constraints for the tissue elements indicating optimization, e.g. minimization, of an elastic potential for the tissue elements, i.e. performs the local step of the local/global solver, and determines the spatial locations which optimize the state equation when the elastic potential corresponds to the local solutions, i.e. performs the global step of the local/global solver. One time step for substantially real-time replication of a surgical operation may correspond to one or more local steps and one or more global steps. As is known to a person skilled in the art, the state equation of motion may generally be solved by solving an optimization problem corresponding to optimizing, e.g. minimizing, the sum of a momentum potential, which corresponds to movement and external forces (here: for the tissue elements), and an elastic potential, which corresponds to internal forces (here: for the tissue elements), i.e. interactions (here: between the tissue elements). The plurality of tissue elements may be in an undeformed configuration or in a deformed configuration. A solution to the state equation may then be considered as correspond to optimizing, e.g. minimizing, elastic deformation, which corresponds to optimizing, e.g. minimizing, the elastic potential. For the local/global solver, as is also known to the person skilled in the art, the elastic potential can be decoupled into a distance measure and a constraint indicator, wherein the latter indicates an undeformed configuration, which may also be indicated as one where the internal constraints for the system are met, and the former indicates the distance between the deformed configuration and the undeformed configuration. Since there may be several possible undeformed configurations, the local step may then be considered as corresponding to determining the undeformed configuration which optimizes, e.g. minimizes, the distance measure. The global step, on the other hand, may be considered as optimizing, e.g. minimizing, the elastic potential under the condition that the undeformed configuration is the one obtained in the local step. The distance measure may be chosen in various ways, for example so that the elastic potential is quadratic for numerical simplicity. Here, the elastic potential can be, for example, quadratic in the spatial locations of the tissue elements and/or the local solutions. The local solutions are auxiliary solutions and are not necessarily directly related to the spatial locations, for example they may have different scaling. The local solutions are obtained, in the local step, when auxiliary spatial locations for the tissue elements are defined in accordance with the one or more constraints for the tissue elements. The local solutions can thereby be considered as states for the tissue elements, where the auxiliary spatial locations satisfy the constraints and optimize, e.g. minimize, a distance to the actual spatial locations for the tissue elements. They can also be considered to correspond to an undeformed configuration for the plurality of tissue elements. Correspondingly, the local step can be considered to involve solving a set of states for the tissue elements corresponding to the spatial locations of the tissue elements when the constraints for the tissue elements are independently satisfied. Each constraint typically corresponds only to a limited number of tissue elements and therefore a limited number of local solutions, allowing notable parallelization of the local step.

The constraints for the tissue elements may comprise or consist of configurational constraints, which can be expressed in terms of one or more spatial locations each corresponding to a tissue element, i.e. as a function of one or more spatial locations for tissue elements. There may be one or more constraints corresponding to each tissue element. For example, the constraints may comprise one or more constraints corresponding to a limited spatial location, such as a fixed spatial location, for a tissue element or a limited distance, such as a fixed distance, between the spatial locations for a pair of tissue elements, defined individually for the pair of tissue elements or generally for any or all tissue elements or pairs of tissue elements. The constraints may also comprise one or more upper and/or lower limits. The constraints are soft constraints since they are not all necessarily satisfied for each of the tissue elements in the global step. However, using the constraints in the local step allows them to be strictly satisfied there to define the local solutions as states where the constraints are satisfied. While the constraints can be used as hard constraints during the local step, the two-part solution by the local/global solver allows them to function as soft constraints for the determination of the spatial locations for the tissue elements or when the local/global solver is considered as a whole. Using the elastic potential corresponding to the local solutions in the global step allows the constraints to indirectly act as soft constraints when determining the spatial locations for the tissue elements. Since the interactions between the tissue elements can be largely or fully described by the elastic potential and since the elastic potential can be determined in terms of the local solutions obtained in the local step, the determination of the spatial locations for the tissue elements in the global step can be greatly simplified in comparison to directly solving the state equation. The determination may even be performed analytically although it should be understood that various numerical optimization, e.g. minimization, methods can also be used, including stochastic optimization. The determination may be considered as determining the deformed configuration for the plurality of tissue elements, when the undeformed contribution corresponds to the set of local solutions for the local step. Using such a local/global solver has been found to provide notable improvement in real-time replication of a tissue. The local step and the global step can be performed repeatedly for substantially real-time replication of a surgical procedure.

Third, receive one or more signals for determining the position of a virtual surgical instrument following the movement of a tracking device. This allows the virtual surgical instrument to be guided by the movement of the tracking device, which is a physical instrument. The virtual surgical instrument may substantially replicate the movement of the tracking device to allow precise and intuitive control of the virtual surgical instrument by the tracking device. When the method is performed in real-time, this step can be performed repeatedly and substantially continuously. This step may be performed one or more times in any combination of before, during and after with respect to any of the other steps.

Fourth, based on the position and, optionally, movement of the virtual surgical instrument with respect to the tissue elements, alter the one or more constraints to indicate that a cut has been made to the tissue with the virtual surgical instrument. This allows the cut to be represented in the model through a soft constraint specifically applicable for indicating behavior of tissue under surgery. As an example, a constraint limiting the distance between the spatial locations for a pair of elements can be adjusted or removed to indicate that the cut has separated the pair of tissue elements. Since the cut affects the constraints in the local step, it indirectly also affects the elastic potential in the global step. As an example, when the global step corresponds to solving a linear system, a system matrix corresponding to the linear system may change in response to the cut.

Below, various embodiments are described, each of which is applicable to the first aspect and/or the second aspect.

In an embodiment, a spatial location for a tissue element corresponds to a density distribution and the one or more constraints comprise a constraint for the density distribution. This allows a continuum to be represented by discrete tissue elements. This, in turn, allows an improved three-dimensional representation specifically for tissue. The density distribution can be defined with respect to an origin of the density distribution, which may correspond to the spatial location of the corresponding tissue element. It may be symmetric or substantially symmetric with respect to the origin, for example spherically symmetric. The density distribution may comprise a part corresponding to a continuous distribution such as a Gaussian distribution. It may, however be defined also so that it differs from zero only within a finite distance from its origin allowing convenient integration into the model. The density distribution may go to zero continuously, which has been found to improve numerical stability of the local/global solver in several situations. Each of the tissue elements may be represented by a density distribution, which may be similar or different. The constraint may, for example, limit the sum of density distributions at one or more points and/or within a region, where the one or more points may correspond to the spatial locations of the tissue elements.

In an embodiment, the set of local solutions is obtained under a constraint indicating that a deformation gradient for the tissue elements for indicating elasticity of the tissue substantially corresponds to a reference deformation gradient, which may be a constant reference deformation gradient. As is known to a person skilled in the art of finite strain theory, any deformation gradient may be represented by a tensor. The deformation gradient may be included in the model, e.g. as a tensor, directly or indirectly, for example as a gradient of displacement. When a spatial location for a tissue element corresponds to a density distribution, the density distribution may indicate how strongly the tissue element affects the deformation gradient. An initial reference deformation gradient or an indication thereof may be determined, for example as part of generating the model, before a cut or any cuts have been made to the tissue, i.e. in the initial state for the tissue elements. The reference deformation gradient may be the deformation gradient corresponding to the initial state. The reference deformation gradient may correspond to an identity matrix. It has been found that using a constraint for a reference deformation gradient markedly improves the robustness of local/global solver under different situations for replicating a surgical procedure, when the density distributions are involved, for example to provide weighting for the interactions between tissue elements. In the absence of such measures or impracticably significant downsizing of the time step for replication of the surgical operation, it has been found that the system of tissue elements may generate an internal energy that grows uncontrollably resulting into a chaotic behavior of the system.

In an embodiment, obtaining the set of local solutions comprises determining the deformation gradient or an indication thereof, wherein rotation is removed from the deformation gradient or the indication thereof. The rotation may be removed with respect to the initial state or the initial reference deformation gradient or indication thereof. This allows the deformation gradient or the indication thereof to be included linearly for the elastic potential. The rotation may be removed using any applicable methods of co-rotated linear elasticity. Numerically, the rotation may be removed, for example, by using a singular value decomposition. Removing the rotation allows the deformation gradient or an indication thereof to be directly used for determining the elastic potential allowing efficient real-time replication of a surgical operation.

In an embodiment, the method comprises determining, or the one or more memories and the computer program code of the apparatus are configured to cause the one or more processors to determine, a neighborhood for a tissue element of the plurality of tissue elements for indicating the group of tissue elements of the plurality of tissue elements that are close enough for interaction with the tissue element. This allows interactions between tissue elements to be included in the model without using a mesh and without separately specifying interactions between individual pairs of tissue elements. The neighborhood for a tissue element can be defined as the group of tissue elements, whose density, which can be represented by the density function, is above a threshold, for example above zero. This can be evaluated, for example, at the spatial location of the tissue element and/or at any point where the density of the tissue element is above a threshold, for example above zero. In particular, the former alternative can be used straightforwardly, as it defines the neighborhood of tissue element as the group of tissue elements whose density is above a threshold, such as zero, at the spatial location of the tissue element. An alternative is to define the neighborhood of tissue elements as the group of tissue elements whose density function overlaps with that of the tissue element. The tissue element, for which the neighborhood is determined, can itself be included in or excluded from the neighborhood. As a person skilled in the art can appreciate, various alternatives for defining the neighborhood exist. As a result of any of these definitions, the neighborhood for a tissue element may rapidly change allowing fast adaptation to shape changes in the tissue.

The method also comprises determining, or the one or more memories and the computer program code of the apparatus are also configured to cause the one or more processors to determine, a constraint for the density distribution for the tissue element as a limitation for cumulative density for the neighborhood for providing incompressibility to the tissue. This allows an improved replication of the tissue as a realistic incompressibility can be included as an intrinsic part of the model. This is particularly applicable for soft tissue and it is directly compatible with fluids so that replication of fluids such as intracellular and/or extracellular fluid, including blood, can be readily included in the model. The cumulative density of the neighborhood for a tissue element can be evaluated, for example, at the spatial location of the tissue element as the sum of density functions for the tissue elements of the neighborhood at that spatial location. The constraint may be, for example, a target density. The constraint may also be an upper or a lower limit for density. A neighborhood can be defined for each tissue element. Each neighborhood may have a constraint for cumulative density, which may be the same or different for different neighborhoods.

In an embodiment, determining the constraint for density distribution comprises determining a scaling factor for scaling the distances between the tissue elements in the neighborhood so that the cumulative density for the neighborhood is limited. This allows the internal pressure arising from interactions between tissue elements to be controlled with a simple constraint corresponding to a scaling factor. In effect, each scaling factor indicates for its corresponding neighborhood whether the density of the neighborhood is too high, or low, and how much the tissue elements of the neighborhood should be moved. A constraint for density distribution corresponding to a scaling factor may be defined for one or more, e.g. for each, of the tissue elements of the plurality of tissue elements.

In an embodiment, the method comprises determining, or the one or more memories and the computer program code of the apparatus are configured to cause the one or more processors to determine, a neighborhood for a tissue element of the plurality of tissue elements for indicating the group of tissue elements of the plurality of tissue elements that are close enough for interaction with the tissue element. Here applies what is disclosed above for the neighborhood. The method also comprises determining, or the one or more memories and the computer program code of the apparatus are also configured to cause the one or more processors to determine, the distances between the tissue elements in the neighborhood for determining weighing coefficients for interaction between the tissue elements. The weighing coefficients can be included in the elastic potential in the local step and/or in the global step. Together with the use of density functions for the tissue elements, the use of weighing coefficients allows an improved replication of the tissue, while simultaneously being solvable by the local/global solver.

In an embodiment, the model is used for causing a haptic feedback to be generated. The haptic feedback allows simulating the interaction between a surgical instrument and a tissue. The tracking device of the apparatus can comprise an actuator for haptic feedback and the apparatus can be arranged to use the model for generating a haptic feedback to the actuator. The haptic feedback may be generated at least partially based on the position of the virtual surgical instrument with respect to a tissue element. The haptic feedback may be generated, for example, in response to a cut being made to the tissue and/or one or more tissue elements interacting with the virtual surgical instrument. The haptic feedback may be generated as physical movement such as vibration.

In an embodiment, altering the one or more constraints to indicate that a cut has been made comprises including one or more additional constraints for a new tissue element generated in response to the cut. The new tissue elements allow increasingly sharp-featured cuts to be made to the tissue. A spatial location for a new tissue element can correspond to a density distribution, which may correspond to a smaller density than the density distribution of the nearest pre-existing tissue element, i.e. one that was included in the model before the cut. One or more new tissue elements may be generated in response to a cut. An initial spatial location for a new tissue element may be determined, for example, by using an initial state deformation gradient of the tissue elements.

According to a third aspect, a computer program product comprises instructions which, when the program is executed by a computer, cause the computer to carry out the method in accordance of the second aspect and/or any of its embodiments alone or in any combination.

The aspects and embodiments disclosed herein may be used for any kind of surgery. Advantageously, they may be adapted for open orthopedics.

A spatial location may correspond to an absolute or a relative spatial location.

It is to be understood that the aspects and embodiments described above may be used in any combination with each other. Several of the aspects and embodiments may be combined together to form a further embodiment of the invention.

## BRIEF DESCRIPTION OF THE DRAWINGS

The accompanying drawings, which are included to provide a further understanding and constitute a part of this specification, illustrate examples and together with the description help to explain the principles of the disclosure. In the drawings:

    **Fig. 1** illustrates an apparatus according to an example,
    **Fig. 2** illustrates a method according to an example,
    **Fig. 3** illustrates a first detailed method according to an example, and
    **Fig. 4** illustrates a second detailed method according to an example.

Like references are used to designate equivalent or at least functionally equivalent parts in the accompanying drawings.

## DETAILED DESCRIPTION

The detailed description provided below in connection with the appended drawings is intended as a description of examples and is not intended to represent the only forms in which the example may be constructed or utilized. However, the same or equivalent functions and structures may be accomplished by different examples.

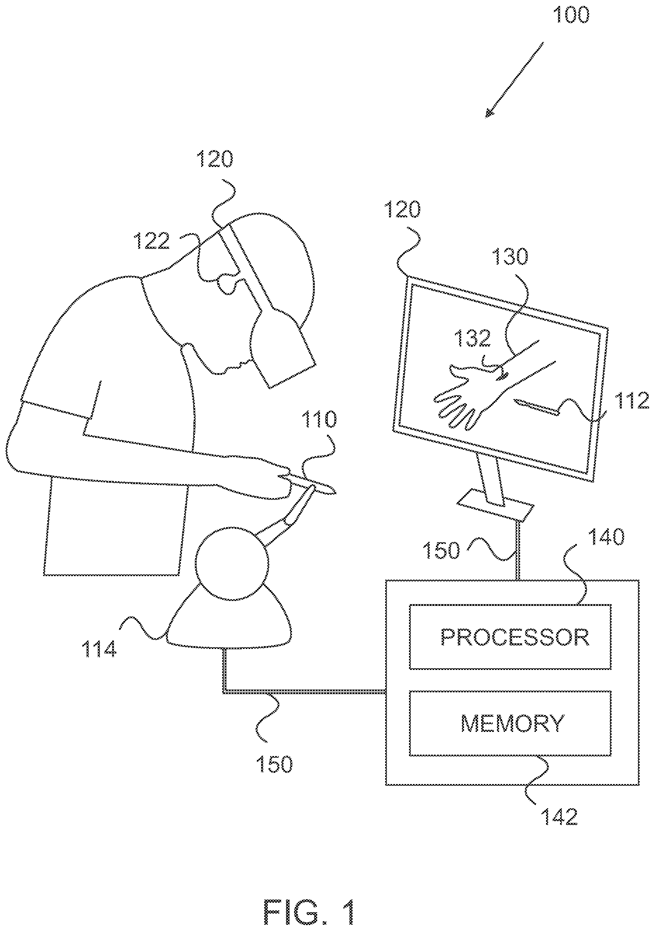

Figure 1 shows an example of an apparatus 100 for surgery preparation. The apparatus 100 comprises a tracking device 110 for representing a surgical instrument. The tracking device 110 may be arranged to move in three dimensions for three-dimensional movement of a virtual surgical instrument 112. The surgical instrument may be a cutting instrument such as a scalpel. The tracking device 110 may comprise a hand-held portion for representing the surgical instrument. For example, the tracking device 110 may comprise a pointed instrument such as a stylus for representing the surgical instrument. Alternatively or additionally, the tracking device 110 may comprise a wearable device such as a glove. Optionally, the tracking device 110 may comprise or be coupled to a base 114. This allows some or all of the electronics for the tracking device 110 to be housed in the base 114. The hand-held portion of the tracking device 110 may be coupled to the base 114 wirelessly and/or through an articulated arm. The hand-held portion may also be self-contained allowing the tracking device 110 to be moved freely without rigid connection to a fixed base 114. The tracking device 110 allows performing simulated movement for a surgical instrument and tracking the movement so that the position, and optionally the orientation, for the surgical instrument can be communicated forward.

The apparatus 100 also comprises a display device 120 for displaying a subject 130 for surgery. The apparatus 100 may also be arranged to use the display device to display the virtual surgical instrument 112. The display device 120 may comprise one or more screens such as a computer screen and/or one or more projectors. Alternatively or additionally, the display device 120 may comprise a headset, such as a virtual reality or an augmented reality headset. This can be used to allow an immersive experience for the user of the apparatus 100 preparing for surgery. The apparatus 100 may further comprise one or more speakers 122, which may be used for example for providing instructions and/or sound for surgery.

The subject 130 for surgery may be, for example, a human body or a part of it, such as a wrist or an ankle. One or more cuts 132, such as incisions, may be displayed on the subject 130 for surgery in response to the virtual surgical instrument 112 cutting the subject 130 for surgery. The apparatus 100 may be arranged to use the display device 120 to simultaneously display the subject 130 for surgery and the virtual surgical instrument 112 so that the person using the apparatus 100 can observe their relative positions. For example, the subject 130 and the virtual surgical instrument may be simultaneously displayed on one screen and/or in the same virtual reality environment. One option is also to display the subject 130 for surgery in an augmented reality environment so that the person using the apparatus 100 can observe the relative position of the subject 130 for surgery and the tracking device 110 representing the surgical instrument.

The apparatus 110 comprises one or more processors 140, which are arranged to receive signals for determining the position, and optionally the orientation of the virtual surgical instrument 112. The signals may be received from the tracking device 110 directly and/or indirectly. The one or more processors 140 may comprise a central processing unit and/or a graphics processing unit. The one or more processors 140 may be arranged to be coupled to the tracking device 110 through one or more connections 150, which may comprise a wireless and/or a wired connection. Similarly, the one or more processors 140 may be arranged to be coupled to the display device 120 through one or more connections 150, which may comprise a wireless and/or a wired connection. The tracking device 110 may comprise one or more actuators for haptic feedback. The one or more actuators may be arranged to provide haptic feedback in response to one or more signals from the one or more processors 140. The haptic feedback may be generated, for example, to a stylus and/or to a wearable device such as a glove.

The apparatus further comprises one or more memories 142, which comprise computer program code. Together, these may be configured to cause the one or more processors 140 to perform any or all of the methods of the subsequent examples.

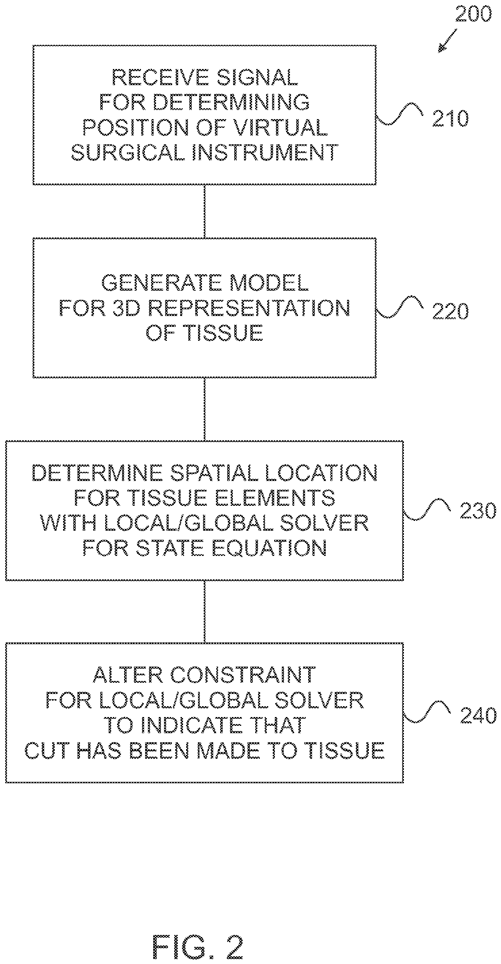

Figure 2 shows an example of a method 200. The method comprises several parts which may be performed independently from each other and/or in any order. The method can be used for replication of a surgical operation in a computerized environment. Consequently, it can be used for surgery preparation, such as training for surgery. The subject for surgery, including a target tissue to be surgically operated can be, for example, selected from a pre-existing database or generated when the replication of the surgical operation is started. While the subject can be any subject, the method has been found to be particularly applicable for real-time replication of human soft tissue.

The method 200 comprises receiving 210 one or more signals for determining the position, and optionally the orientation, of a virtual surgical instrument 112. The position, and optionally the orientation, of the virtual surgical instrument 112 may follow movement of a tracking device 110 representing a surgical instrument. Following the movement may include following changes in position and/or orientation.

The method 200 also comprises generating 220 a model for three-dimensional representation of a tissue corresponding to the subject 130 for surgery. The model comprises a plurality of tissue elements, which are arranged to together represent the tissue. In an advantageous embodiment, the tissue is soft tissue such as fat tissue, skin tissue, muscle tissue and/or ligaments. The tissue elements may be point-like elements in the sense that they have a single well-defined spatial location, which indicates the position of the tissue element. Generating the model may comprise generating one or more relations between the tissue elements, for example one or more relations between two adjacent tissue elements. While the method may also be applied to meshed structure of tissue elements, using a meshless structure of tissue elements provides particular advantages for the surgery application as it allows producing cuts in the tissue without having to redefine the mesh. An initial state may also be determined for the plurality of tissue elements. The initial state may correspond to a state before a cut or any cuts have been made to the tissue. The initial state may comprise the spatial locations of the tissue elements and/or any values derivable therefrom.

The method 200 also comprises determining 230 a spatial location for each of the tissue elements with a local/global solver for a state equation of motion for the tissue elements. The local/global solver is arranged to provide a solution (hereafter "the solution"), which can be an approximate solution, to the state equation of motion for the tissue elements for determination of the spatial locations of the tissue elements. The local/global solver allows providing the solution by alternatingly performing a parallelizable local step and a global step utilizing local solutions obtained from the local step. Similarly, the steps can be alternated so that each global step is followed by a local step. The local/global solver alternatingly, in a local step, obtains a set of local solutions under one or more constraints for the tissue elements indicating optimization, e.g. minimization, of an elastic potential for the tissue elements and, in a global step, determines the spatial locations which optimize the state equation when the elastic potential corresponds to the local solutions. This allows turning the difficult problem of solving the full state equation into a constantly evolving optimization problem, where individual steps may even be analytically solved. The local/global solver may utilize Projective Dynamics for providing the solution. Examples of Projective Dynamics are given, for example in "Bouaziz et al. - Projective Dynamics: Fusing Constraint Projections for Fast Simulation; ACM Transactions on Graphics, 33-4, 2014" (here also "Bouaziz"). While the examples in Bouaziz utilize meshes, it is emphasized that in the present invention point-like tissue elements may also be used allowing particularly advantageous application for surgery. Even then, the local solutions may correspond to projections of Projective Dynamics. The local/global solver may be arranged to alternate between projection and distance optimization, e.g. minimization.

The state equation of motion for the tissue elements, for which the solution is provided, can be expressed as defining the spatial location and velocity of each tissue element, which spatial location and/or velocity may change over time. This allows the tissue elements to follow general laws of motion, such as the Newton's laws of motion. The state equation can be expressed as a matrix equation. The state equation may be defined in terms of a mass matrix $\mathbf{M}$, which describes how the velocities $\mathbf{v}$ of the tissue elements at spatial locations $\mathbf{x}$ is affected by forces internal $\mathbf{f}_{int}(\mathbf{x})$ and/or external $\mathbf{f}_{ext}$ to the tissue, for example through the equation

$$\mathbf{v}_{i+1} = \mathbf{v}_i + h\mathbf{M}^{-1}(\mathbf{f}_{int}(\mathbf{x}_{i+1})+\mathbf{f}_{ext}), \qquad (1)$$

where $\mathbf{M}^{-1}$ is the inverse of matrix $\mathbf{M}$ and the values at subsequent time steps (*i* and *i+1*) are indicated as subscripts, whereas the length of the time step is denoted as $h$ (this notation will also be used below). In general, the mass matrix may be constant so that it remains the same from one step to another. However, the mass matrix may be altered in response tissue elements being added to and/or subtracted from the model for three-dimensional representation of a tissue. As an example, the state of the tissue elements at subsequent time steps may then be related through an equation of the form

$$\mathbf{M}(\mathbf{x}_{i+1}-\mathbf{x}_i-h\mathbf{v}_i)=h^2(\mathbf{f}_{int}(\mathbf{x}_{i+1})+\mathbf{f}_{ext}). \qquad (2)$$

Such an equation can be converted (examples in Bouaziz, particularly in section 3.1, and "Martin et al. - Example-based Elastic Materials; ACM Transactions on Graphics, 30-4, 2011)" into an optimization problem of the type

$$\mathbf{x}_{i+1} = \text{argmin} (\mathbf{V}_{mom} + \mathbf{V}_{el}), \qquad (3)$$

where the arguments of the minima is determined for the spatial locations $\mathbf{x}$ and terms that either correspond to the external forces or are proportional to the mass matrix can be contained in a momentum potential $\mathbf{V}_{mom}$, whereas terms corresponding to the internal forces of the tissue can be contained in an elastic potential $\mathbf{V}_{el}$.

In general, the solution to the state equation of motion for the tissue elements can be provided by solving an optimization problem, where the sum of a momentum potential and an elastic potential is optimized, e.g. minimized, with respect to the spatial locations $\mathbf{x}$ of the tissue elements. The elastic potential then corresponds to the internal forces of the tissue. The momentum potential then becomes the remaining part, or the part corresponding to the external forces and the mass matrix. The elastic potential can, in turn, be defined as a sum of a distance measure and an indicator function, the latter of which becomes infinite if a set of constraints is not satisfied. Examples are given in Bouaziz, particularly in section 3.2. While the exact form of the elastic potential may vary, one example is the elastic potential having a quadratic distance measure, examples of which are also given in Bouaziz, particularly in section 3.2. It is noted that while in Bouaziz constant matrices $\mathbf{A}$ and $\mathbf{B}$ are used in the distance measure, here they can change, for example in response to tissue elements being separated from each other. Correspondingly, the elastic potential may be changed in response to tissue elements being separated from each other, allowing surgical cuts to be efficiently made to the tissue.

In general, the elastic potential can be determined such that it decouples a distance measure and a constraint manifold. This allows the local/global solver to parallelize the optimization problem for providing the solution to the state equation of motion for the tissue elements. Examples are given in Bouaziz, particularly in section 3.3. The local step may even be arranged to correspond to optimization, e.g. minimization, of the elastic potential alone. The elastic potential can be optimized with respect to auxiliary variables to yield local solutions. In the local step, local solutions can be obtained due to one or more constraints defined for the tissue elements. The one or more constraints may comprise one or more constraints representing interaction between tissue elements, for example an interaction between two tissue elements, which can be expressed as a limitation for the relative spatial location between the two tissue elements. Correspondingly, the one or more constraints may comprise one or more constraints representing tissue elements being connected to each other. This allows a cut in the tissue to be represented by an alteration of the one or more constraints, in particular those representing tissue elements being connected to each other, for example by removing one or more constraints representing tissue elements being connected to each other. The one or more constraints can be defined to allow parallelization of the optimization. For example, such constraints may comprise constraints for defining absolute and/or relative spatial locations of one or more tissue elements for par-allelizing the optimization of the elastic potential. For example, optimizing the elastic potential with respect to a single tissue element, whose interaction with other tissue elements is constrained only to a limited number of other tissue elements allows paral-lelizing the local step, so that the optimization in the local step is markedly simplified from that of the full optimization problem.

In the global step, the local solutions can then be used to simplify the many-body problem of internal interactions between the tissue elements. In particular, the local/global solver can be arranged to, in the global step, determine the spatial locations which optimize the state equation when the elastic potential corresponds to the local solutions. As an example, this may correspond to solving Equation 3, when the local solutions are inserted into the elastic potential. In some embodiments, for example with a quadratic distance measure of the elastic potential, the local/global solver can be arranged to be able to provide the solution with a single linear solve. It is noted that the local solutions in the elastic potential can make the global optimization problem analytically solvable. The system matrix in the global step may change, for example in response to tissue elements being separated from each other. A solution from the global step may directly correspond to the solution of the local/global solver. It may be provided numerically. Applicable methods are, for example, Jacobi method, Gauss-Seidel method and any form of conjugate gradient method.

The method 200 may further comprise altering 240 the one or more constraints to indicate that a cut 132 has been made to the tissue with the virtual surgical instrument 112. This can be done based on the position, and optionally the orientation and/or velocity, of the virtual surgical instrument 112 with respect to the tissue elements. The cut 132 can be made, and also be transmitted for visualization, substantially in real-time, allowing the method to be used for surgery preparation. In response to the cut 132, haptic feedback can also be generated. For example, the method may comprise a step where the model is used to generate a haptic feedback, for example by an actuator for haptic feedback, which may be comprised in the tracking device 110. The haptic feedback may be caused to be generated based on one or more changes for the tissue elements, for example in response to a cut 132 and/or a change in spatial location of one or more tissue elements. Alternatively or additionally, it may be caused to be generated in response to relative movement of the virtual surgical instrument 112 and one or more tissue elements, for example in response to the virtual surgical instrument 112 being within a threshold distance from one or more tissue elements. The haptic feedback may comprise any combination a force, a vibration and a motion.

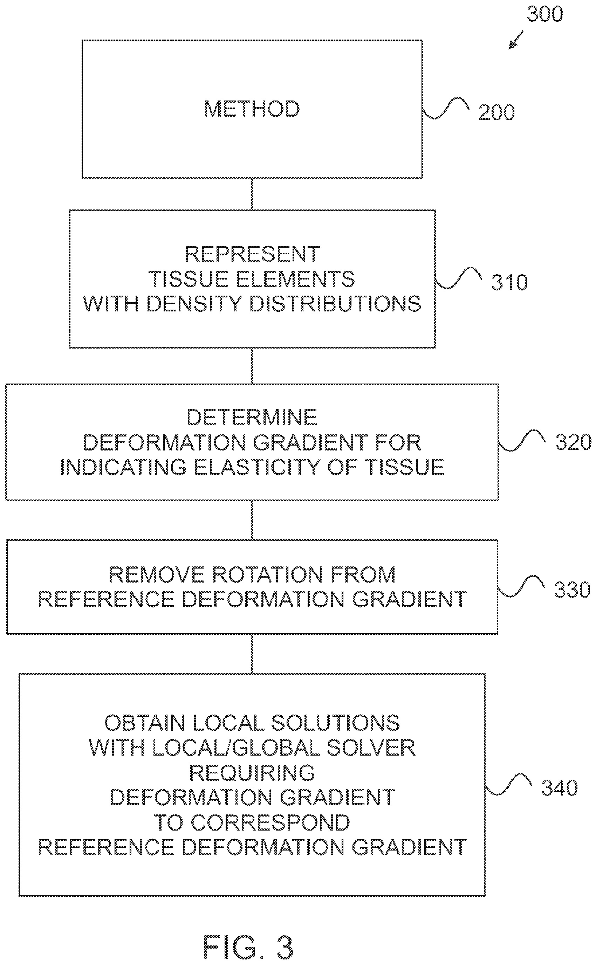

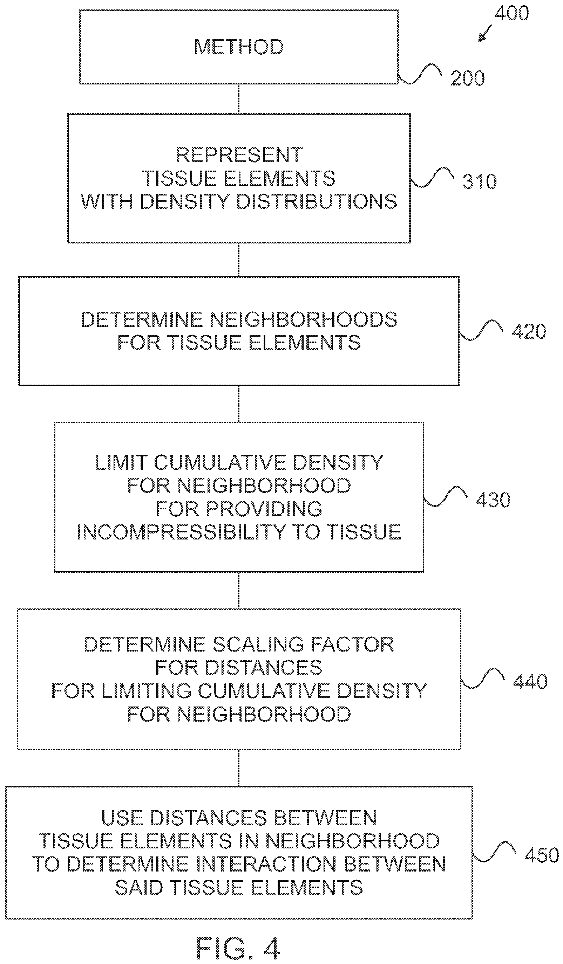

Figures 3 and 4 show two detailed examples of methods, which can be performed independently from each other or as a combined method. The apparatus 100 and/or a computer program product may be configured to perform either or both of the methods. In accordance with these examples, a method may comprise any or all features of the methods described above together with the following additional features, which may be included in any suitable order within a composite method.

A feature common for both cases is that tissue elements can be represented with density distributions. This allows the tissue to be replicated as a continuum instead of spatially discrete tissue elements. However, the tissue elements continue to be characterized by their spatial location and the spatial location for a tissue element thereby corresponds to the density distribution for the tissue element. Each tissue element can therefore correspond to both a spatial location and a density distribution. The spatial location for the tissue element may correspond, for example, a center point and/or a symmetry point for the density distribution. For example, the density distribution may be, at least substantially, rotationally symmetric or spherically symmetric with respect to the spatial location for the tissue element. However, other shapes for the density distribution are also possible. In any case, the density distribution is non-vanishing, or non-zero, also outside the spatial location for the tissue element. The density distribution has a total density, which can be determined as a sum or an integral of the density distribution over space. The density distribution can have a continuous part but, optionally, it can also have one or more discontinuous parts. The density distribution may vanish, or be zero, above a threshold distance from the spatial location. This allows the interactions between tissue elements to be limited to close neighbors. The density distribution may go to zero continuously, which can improve the stability of the local/global solver. The density distribution for two or more, or even all, tissue elements for the tissue may have at least substantially the same shape, allowing a simple definition of the tissue. However, density distributions of two or more different shapes can also be used to allow different types of tissue elements. Similarly, the total density of the density distribution may be constant for the tissue elements for the tissue or one or more tissue elements may correspond to a density distribution having a different total density. The density distribution may, for example, comprise a Gaussian shape, which may be limited away from the spatial location for the corresponding tissue element. The peak of the density distribution may correspond to the spatial location for the corresponding tissue element.

Using both a distinct spatial location and a density distribution for a tissue element allows the tissue element to have a single well-defined spatial location while simultaneously simulating a continuum. In particular, maintaining the single well-defined spatial location allows the local/global solver to be used as described above. In addition, interactions between tissue elements can be determined using the one or more constraints for the local/global solver. The one or more constraints may comprise one or more constraints for the density distribution. These constraints may be, at least partially, common to several or all tissue elements. An example of a constraint is a threshold value for density, for example for cumulative density at a spatial position, such as the spatial location of a tissue element.

The use of density functions further allows integrating fluids into the method conveniently, which is a specifically advantageous feature for surgery. This further allows an improved process of forming a tissue by creating an empty space, causing tissue elements with liquid-like interactions fill the space, at least partially, and when the tissue elements have accumulated into form, create an elastic structure based on their spatial locations. This way, the distances between the tissue elements are optimized by liquid dynamics. Such a structure could be, for example, fat tissue. Consequently, a method for generating a tissue for surgery preparation can thus be provided.

Figure 3 shows an example of a method 300, a combination of features which has been found to bring realistic elasticity into the tissue to allow improved real-time replication of a surgical operation.

Here, any method 200 described above has tissue elements represented 310 with density distributions. In addition, a deformation gradient or an indication of a deformation gradient (both of us referred below as "the deformation gradient") is determined 320. Here, the deformation gradient indicates elasticity of the tissue and it can be formulated in accordance with the principles of strain theory or finite strain theory in particular. The deformation gradient can be used to indicate how the deformation of the tissue changes spatially. It can indicate the spatial change of deformation between an earlier state and a later state for the tissue elements. The deformation gradient may be represented with a matrix, or a tensor. It may act as a transformation matrix or tensor which transforms an infinitesimal line element from an initial position to the current position. Examples of a deformation gradient can be seen in "Peer et al. - An Implicit SPH Formulation for Incompressible Linearly Elastic Solids: Implicit Elastic SPH Solids; Computer Graphics Forum 37-6, 2017" (here also "Peer"), particularly in sections 3.1. and 3.3. of the reference. Accordingly, the deformation gradient can be a Smoothed Particle Hydrodynamics deformation gradient. As an indication of a deformation gradient, a displacement gradient or any other construct providing the corresponding indication of the elasticity of the tissue can be used.

Importantly, the set of local solutions for the local/global solver can be obtained 340 under a constraint that the deformation gradient substantially corresponds to a reference deformation gradient, which may be a constant reference deformation gradient. In particular, the reference deformation gradient may at least substantially correspond to a previously determined state for the tissue elements, such as the initial state for the tissue elements. The previously determined state may be a state for the tissue elements which has been determined before a cut 132 has been made to the tissue with the virtual surgical instrument 112. This can be achieved by having the reference deformation gradient correspond to an identity matrix. The previously determined state may also be a state for the tissue elements prior to or at the beginning of the local step. Correspondingly, the deformation gradient or the reference deformation gradient may change during the replication of the surgical operation, for example in response to a cut 132 being made by the virtual surgical instrument 112. Even in this case, the deformation gradient can still be made to correspond to a reference deformation gradient over a single execution of the local step, for example by excluding any tissue elements separated by the cut 132 from the deformation gradient and the reference deformation gradient is determined anew without the excluded tissue element or elements. Irrespective of whether the previously determined state is the initial state or a later state, making the deformation gradient correspond to the reference deformation gradient over a local step has been found to allow improved real-time replication of a surgical operation, particularly when density functions are used for tissue elements. Using the constraint as one of the one or more constraints in the local step allows accommodating the constraint for the local/global solver.

The solution from the local/global solver may be provided utilizing the linear elasticity approximation. Also, it has been found that the reference deformation gradient can be co-rotated to significantly improve the real-time performance of the method. Examples of a co-rotated deformation gradient are shown in Peer, particularly in section 3.3. of the document. Removing 330 rotation from the reference deformation gradient has been found to be compatible with the local/global solver. It is noted that while a standard method for deformable elastic solids is to remove rotation from a system with a Cauchy-Green deformation tensor, here the rotation can be removed without squaring the deformation gradient. Instead, the rotation removal can be linear with respect to deformation gradient, for example using a rotation extraction method corresponding to that shown in Peer. The rotation can be removed so that the deformation gradient can directly be used as a linear strain or deformation tensor.

Figure 4 shows an example of a method 400, a combination of features which has been found to bring realistic incompressibility into the tissue to allow improved real-time replication of a surgical operation.

Here, any method 200, 300 described above has tissue elements represented 310 with density distributions. In addition, a neighborhood can be determined 420 for one or more tissue elements. The neighborhood indicates the group of tissue elements (hereafter also "neighboring tissue elements") that are close enough for interaction with the tissue element for which the neighborhood is defined (hereafter also "main tissue element"). As a matter of definition, the neighborhood may either include or exclude the tissue element with respect to which the neighborhood is determined. Correspondingly the group may consist of zero, one or more tissue elements. The neighborhood for a tissue element may be determined based on a threshold distance, for example so that neighboring tissue elements are those whose spatial location is within the threshold distance from the spatial location for the main tissue element. Any or all neighborhoods for tissue elements may be determined anew for subsequent local steps. This can be used to provide incompressibility after a cut 132 has been made to the tissue since the model can now, in real-time, determine the changing closeness relationships for tissue elements. A neighborhood may be defined for each tissue element so that each tissue element is the main tissue element for a neighborhood. Depending on the configuration of the tissue and the definition of the neighborhood, it may be possible, at least in some situations, that one or more tissue elements are not part of any neighborhoods. In addition to the matter of incompressibility, using the neighborhoods allows the tissue to be replicated without a separate collision detection system, significantly improving the real-time performance of the method. It is noted that when the neighborhoods are used, the initial state for the tissue elements needs not necessarily be defined or used. Instead, the reference deformation gradient may be separately defined for each neighborhood. These neighborhoods related by elasticity may change, even solely, in response to a cut 132. In contrast, neighborhoods related by incompressibility may change freely between time steps. Also, the deformation gradients for the neighborhoods may be made to correspond to reference deformation gradients, such as constant reference deformation gradients, for the neighborhoods over the local step, as described above, for example so that any or all reference deformation gradients for the neighborhood correspond to identity matrices. This can provide the abovementioned effect of robustness even for neighborhoods changing from one local step to another. It is noted that since the neighborhoods may change for subsequent local steps, the system matrix for subsequent global steps may change correspondingly.

A constraint for the cumulative density for the neighborhood can then be determined 430 to provide incompressibility to the tissue. The constraint can be used as one of the one or more constraints for the tissue elements in the local step. The cumulative density for the neighborhood can be defined for some or all spatial points of the neighborhood. It has been found that an effective way can be to actually define it for a single point within the neighborhood, which can be the spatial location for the main tissue element. As an example of a simple and effective constraint, the cumulative density for the neighborhood at the spatial location of the tissue element can be limited, for example with a target value, an upper boundary or a lower boundary. Generally, the cumulative density can include a density for each of the neighboring tissue elements, e.g. as a sum density.

As another simple and effective constraint, it has been found that a scaling factor can be determined 440 for limiting the cumulative density. The scaling factor may be determined as the value with which distances of the neighboring tissue elements from the main tissue element need to be multiplied to have a threshold cumulative density for the neighborhood, for example at the spatial location for the main tissue element. The scaling factor may be determined in the local step. The scaling factor may be used in the local and/or global in different ways. One reliable and stable way to do this is to determine an instantaneous deformation gradient for the neighborhood in a similar way to how it is done for the reference deformation gradients. This instantaneous deformation gradient can then be multiplied by the scaling factor and the product can be used as the local solution, to which the global step will try to match the unscaled instantaneous deformation gradient. This may be performed with rotation extraction, for example as for the case of elasticity, but also without, since the neighborhoods related by incompressibility are free to change between time steps. This allows a very efficient way for providing incompressibility to the tissue. The scaling factor can be used to increase the likelihood that, during a single iteration round, the local/global solver moves those tissue elements which have the largest effect on the density of the neighborhoods.

By using density distributions that are functions of distance from the elements, the distances within a neighborhood can also be used to determine interaction strength between tissue elements. Based on the distance between the spatial locations of two tissue elements, a weighing coefficient may be determined for interaction. Decreasing distance between two tissue elements can correspond to a larger overlap in density distributions and, thus, a stronger interaction, which may be indicated by a larger weighing coefficient. The distances may be determined within a neighborhood with respect to the main tissue element so that the distance for each of the neighboring tissue element with respect to the main tissue element is determined. The weighing coefficient may be determined in the local step. The weighing coefficient may be used in the local and/or global step to weight the contributions of the tissue elements to the deformation gradients, for example based on how much their density distributions overlap or by some other indication for how strongly they interact.

The apparatus as described above may be implemented in software, hardware, application logic or a combination of software, hardware and application logic. The application logic, software or instruction set may be maintained on any one of various conventional computer-readable media. A "computer-readable medium" may be any media or means that can contain, store, communicate, propagate or transport the instructions for use by or in connection with an instruction execution system, apparatus, or device, such as a computer. A computer-readable medium may comprise a computer-readable storage medium that may be any media or means that can contain or store the instructions for use by or in connection with an instruction execution system, apparatus, or device, such as a computer. The examples can store information relating to various processes described herein. This information can be stored in one or more memories, such as a hard disk, optical disk, magneto-optical disk, RAM, and the like. One or more databases can store the information used to implement the embodiments. The databases can be organized using data structures (e.g., records, tables, arrays, fields, graphs, trees, lists, and the like) included in one or more memories or storage devices listed herein. The databases may be located on one or more devices comprising local and/or remote devices such as servers. The processes described with respect to the embodiments can include appropriate data structures for storing data collected and/or generated by the processes of the devices and subsystems of the embodiments in one or more databases.

All or a portion of the embodiments can be implemented using one or more general purpose processors, microprocessors, digital signal processors, micro-controllers, and the like, programmed according to the teachings of the embodiments, as will be appreciated by those skilled in the computer and/or software art(s). Appropriate software can be readily prepared by programmers of ordinary skill based on the teachings of the embodiments, as will be appreciated by those skilled in the software art. In addition, the embodiments can be implemented by the preparation of application-specific integrated circuits or by interconnecting an appropriate network of conventional component circuits, as will be appreciated by those skilled in the electrical art(s). Thus, the embodiments are not limited to any specific combination of hardware and/or software.

The different functions discussed herein may be performed in a different order and/or concurrently with each other.

Any range or device value given herein may be extended or altered without losing the effect sought, unless indicated otherwise. Also any example may be combined with another example unless explicitly disallowed.

Although the subject matter has been described in language specific to structural features and/or acts, it is to be understood that the subject matter defined in the appended claims is not necessarily limited to the specific features or acts described above. Rather, the specific features and acts described above are disclosed as examples of implementing the claims and other equivalent features and acts are intended to be within the scope of the claims.

It will be understood that the benefits and advantages described above may relate to one embodiment or may relate to several embodiments. The embodiments are not limited to those that solve any or all of the stated problems or those that have any or all of the stated benefits and advantages. It will further be understood that reference to 'an' item may refer to one or more of those items.

The term 'comprising' is used herein to mean including the method, blocks or elements identified, but that such blocks or elements do not comprise an exclusive list and a method or apparatus may contain additional blocks or elements.

## Claims

1. An apparatus (100) for surgery preparation, the apparatus (100) comprising:

   a tracking device (110) for representing a surgical instrument (112);
   a display device (120) for displaying a subject for surgery (130);
   one or more processors (140) arranged to receive signals corresponding to the tracking device (110) to determine the position of a virtual surgical instrument (112) following the movement of the tracking device (110); and
   one or more memories (142) comprising computer program code, the one or more memories (142) and the computer program code configured to cause the one or more processors (140) to:

   generate a model for three-dimensional representation of a tissue corresponding to the subject for surgery (130), the model comprising plurality of tissue elements arranged to together represent the tissue; based on the position of the virtual surgical instrument (112) with respect to the tissue elements, alter the one or more constraints to indicate that a cut (132) has been made to the tissue (240) with the virtual surgical instrument (112); and **characterized in that** the one or more processors (140) is further configured to determine a spatial location for each of the tissue elements with a local/global solver for a state equation of motion for the tissue elements (230), which local/global solver alternatingly obtains a set of local solutions under one or more constraints for the tissue elements indicating optimization of an elastic potential for the tissue elements and determines the spatial locations which optimize the state equation when the elastic potential corresponds to the local solutions.

2. The apparatus according to claim 1, wherein a spatial location for a tissue element corresponds to a density distribution and the one or more constraints comprise a constraint for the density distribution.

3. The apparatus according to claim 2, wherein the set of local solutions is obtained under a constraint indicating that a deformation gradient for the tissue elements for indicating elasticity of the tissue substantially corresponds to a reference deformation gradient.

4. The apparatus according to claim 3, wherein obtaining the set of local solutions comprises determining the deformation gradient or an indication thereof, wherein rotation is removed from the reference deformation gradient or the indication thereof.

**5.** The apparatus according to any of claims 2-4, wherein the one or more memories and the computer program code are configured to cause the one or more processors to:

determine a neighborhood for a tissue element of the plurality of tissue elements for indicating the group of tissue elements of the plurality of tissue elements that are close enough for interaction with the tissue element; and determine a constraint for the density distribution for the tissue element as a limitation for cumulative density for the neighborhood for providing incompressibility to the tissue.

**6.** The apparatus according to claim 5, wherein determining the constraint for density distribution comprises determining a scaling factor for scaling the distances between the tissue elements in the neighborhood so that the cumulative density for the neighborhood is limited.

**7.** The apparatus according to any of claims 2-6, wherein the one or more memories and the computer program code are configured to cause the one or more processors to:

determine a neighborhood for a tissue element of the plurality of tissue elements for indicating the group of tissue elements of the plurality of tissue elements that are close enough for interaction with the tissue element; and determine the distances between the tissue elements in the neighborhood for determining weighing coefficients for interaction between the tissue elements.

**8.** The apparatus according to any preceding claim, wherein the tracking device comprises an actuator for haptic feedback and the apparatus is arranged to use the model for generating a haptic feedback to the actuator.

**9.** A method comprising:

generating a model for three-dimensional representation of a tissue corresponding to a subject for surgery (130), the model comprising plurality of tissue elements arranged to together represent the tissue; based on the position of the virtual surgical instrument (112) with respect to the tissue elements, alter the one or more constraints to indicate that a cut (132) has been made to the tissue (240) with the virtual surgical instrument (112); and **characterized in that**
determining a spatial location for each of the tissue elements with a local/global solver for a state equation of motion for the tissue elements (230), which local/global solver alternatingly obtains a set of local solutions under one or more constraints for the tissue elements indicating optimization of an elastic potential for the tissue elements and determines the spatial locations which optimize the state equation when the elastic potential corresponds to the local solutions;
receiving one or more signals for determining the position of a virtual surgical instrument (112) following the movement of a tracking device (110).

**10.** The method according to claim 9, wherein a spatial location corresponds for a tissue element corresponds to a density distribution and the one or more constraints comprise a constraint for the density distribution.

**11.** The method according to claim 10, wherein the set of local solutions is obtained under a constraint indicating that a deformation gradient for the tissue elements for indicating elasticity of the tissue substantially corresponds to a reference deformation gradient and wherein obtaining the set of local solutions comprises determining the deformation gradient or an indication thereof, wherein rotation is removed from the reference deformation gradient or the indication thereof.

**12.** The method according to any of claims 10-11, comprising:

determining a neighborhood for a tissue element of the plurality of tissue elements for indicating the group of tissue elements of the plurality of tissue elements that are close enough for interaction with the tissue element; and determining a constraint for the density distribution for the tissue element as a limitation for cumulative density for the neighborhood for providing incompressibility to the tissue.

**13.** The method according to any of claims 10-12, comprising:

determining a neighborhood for a tissue element of the plurality of tissue elements for indicating the group of tissue elements of the plurality of tissue elements that are close enough for interaction with the tissue element; and determining the distances between the tissue elements in the neighborhood for determining weighing coefficients for interaction between the tissue elements.

14. The method according to any of claims 9-13, comprising using the model for causing a haptic feedback to be generated.

15. A computer program product comprising instructions which, when the program is executed by a computer, cause the computer to carry out the method of any of claims 9-14.

**Patentansprüche**

1. Vorrichtung (100) zur Operationsvorbereitung, wobei die Vorrichtung (100) umfasst: eine Verfolgungsvorrichtung (110) zum Darstellen eines chirurgischen Instruments (112); eine Anzeigevorrichtung (120) zum Anzeigen eines zu operierenden Objekts (130); einen oder mehrere Prozessoren (140), die so angeordnet sind, dass sie einen oder mehrere Prozessoren (140), die angeordnet sind, um Signale zu empfangen, die der Verfolgungsvorrichtung (110) entsprechen, um die Position eines virtuellen chirurgischen Instruments (112) im Anschluss an die Bewegung der Verfolgungsvorrichtung (110) zu bestimmen; und einen oder mehrere Speicher (142), die Computerprogrammcode umfassen, wobei der eine oder die mehreren Speicher (142) und der Computerprogrammcode konfiguriert sind, um den einen oder die mehreren Prozessoren (140) zu veranlassen: Erzeugen eines Modells zur dreidimensionalen Darstellung eines Gewebes, das dem zu operierenden Subjekt (130) entspricht, wobei das Modell eine Vielzahl von Gewebeelementen umfasst, die so angeordnet sind, dass sie zusammen das Gewebe darstellen; Ändern der einen oder mehreren Beschränkungen basierend auf der Position des virtuellen chirurgischen Instruments (112) in Bezug auf die Gewebeelemente, um anzuzeigen, dass mit dem virtuellen chirurgischen Instrument (112) ein Schnitt (132) an dem Gewebe (240) vorgenommen worden ist; und **dadurch gekennzeichnet, dass** der eine oder die mehreren Prozessoren (140) ferner so konfiguriert sind, dass sie einen räumlichen Ort für jedes der Gewebeelemente mit einem lokalen/globalen Löser für eine Zustandsgleichung der Bewegung für die Gewebeelemente (230) bestimmen, wobei der lokale/globale Löser abwechselnd einen Satz lokaler Lösungen unter einer oder mehreren Beschränkungen für die Gewebeelemente erhält, die eine Optimierung eines elastischen Potentials für die Gewebeelemente anzeigen, und die räumlichen Orte bestimmt, die die Zustandsgleichung optimieren, wenn das elastische Potential den lokalen Lösungen entspricht.

2. Vorrichtung nach Anspruch 1, wobei eine räumliche Position für ein Gewebeelement einer Dichteverteilung entspricht und die eine oder mehreren Beschränkungen eine Beschränkung für die Dichteverteilung umfassen.

3. Vorrichtung nach Anspruch 2, wobei der Satz lokaler Lösungen unter einer Beschränkung erhalten wird, welche angibt, dass ein Verformungsgradient für die Gewebeelemente zur Angabe der Elastizität des Gewebes im Wesentlichen einem Referenzverformungsgradienten entspricht.

4. Vorrichtung nach Anspruch 3, wobei das Erhalten des Satzes lokaler Lösungen das Bestimmen des Verformungsgradienten oder eines Hinweises darauf umfasst, wobei die Drehung aus dem Referenzverformungsgradienten oder dem Hinweis darauf entfernt wird.

5. Vorrichtung nach einem der Ansprüche 2 bis 4, wobei der eine oder die mehreren Speicher und der Computerprogrammcode so konfiguriert sind, dass sie den einen oder die mehreren Prozessoren veranlassen:

   Bestimmen einer Nachbarschaft für ein Gewebeelement aus der Vielzahl von Gewebeelementen, um die Gruppe von Gewebeelementen aus der Vielzahl von Gewebeelementen anzuzeigen, die nahe genug für eine Wechselwirkung mit dem Gewebeelement sind; und
   Bestimmen einer Beschränkung für die Dichteverteilung für das Gewebeelement als eine Begrenzung der kumulativen Dichte für die Nachbarschaft, um dem Gewebe Inkompressibilität zu verleihen.

6. Vorrichtung nach Anspruch 5, wobei die Bestimmung der Beschränkung für die Dichteverteilung die Bestimmung eines Skalierungsfaktors zur Skalierung der Abstände zwischen den Gewebeelementen in der Nachbarschaft umfasst, so dass die kumulative Dichte für die Nachbarschaft begrenzt ist.

7. Vorrichtung nach einem der Ansprüche 2-6, wobei der eine oder die mehreren Speicher und der Computerprogrammcode so konfiguriert sind, dass sie den einen oder die mehreren Prozessoren dazu veranlassen:

Bestimmen einer Nachbarschaft für ein Gewebeelement aus der Vielzahl von Gewebeelementen, um die Gruppe von Gewebeelementen aus der Vielzahl von Gewebeelementen anzuzeigen, die nahe genug für eine Wechselwirkung mit dem Gewebeelement sind; und
Bestimmen der Abstände zwischen den Gewebeelementen in der Nachbarschaft, um Wiegekoeffizienten für die Interaktion zwischen den Gewebeelementen zu ermitteln.

8. Vorrichtung nach einem der vorhergehenden Ansprüche, wobei die Verfolgungsvorrichtung einen Aktor für haptisches Feedback umfasst und die Vorrichtung so eingerichtet ist, dass sie das Modell zur Erzeugung eines haptischen Feedbacks für den Aktor verwendet.

9. Verfahren, umfassend:

Erzeugen eines Modells zur dreidimensionalen Darstellung eines Gewebes, das einem zu operierenden Subjekt (130) entspricht, wobei das Modell eine Vielzahl von Gewebeelementen umfasst, die so angeordnet sind, dass sie zusammen das Gewebe darstellen; Ändern der einen oder mehreren Beschränkungen basierend auf der Position des virtuellen chirurgischen Instruments (112) in Bezug auf die Gewebeelemente, um anzuzeigen, dass mit dem virtuellen chirurgischen Instrument (112) ein Schnitt (132) an dem Gewebe (240) vorgenommen wurde; und **gekennzeichnet durch**
Bestimmen eines räumlichen Ortes für jedes der Gewebeelemente mit einem lokalen/globalen Löser für eine Zustandsgleichung der Bewegung für die Gewebeelemente (230), wobei der lokale/globale Löser abwechselnd einen Satz lokaler Lösungen unter einer oder mehreren Beschränkungen für die Gewebeelemente erhält, die eine Optimierung eines elastischen Potentials für die Gewebeelemente anzeigen, und die räumlichen Orte bestimmt, die die Zustandsgleichung optimieren, wenn das elastische Potential den lokalen Lösungen entspricht; Empfangen eines oder mehrerer Signale zur Bestimmung der Position eines virtuellen chirurgischen Instruments (112), das der Bewegung einer Verfolgungsvorrichtung (110) folgt.

10. Verfahren nach Anspruch 9, wobei ein räumlicher Ort für ein Gewebeelement einer Dichteverteilung entspricht und die eine oder mehreren Beschränkungen eine Beschränkung für die Dichteverteilung umfassen.

11. Verfahren nach Anspruch 10, wobei der Satz lokaler Lösungen unter einer Beschränkung erhalten wird, die angibt, dass ein Verformungsgradient für die Gewebeelemente zur Anzeige der Elastizität des Gewebes im Wesentlichen einem Referenzverformungsgradienten entspricht, und wobei das Erhalten des Satzes lokaler Lösungen das Bestimmen des Verformungsgradienten oder einer Angabe davon umfasst, wobei die Drehung aus dem Referenzverformungsgradienten oder der Angabe davon entfernt wird.

12. Verfahren nach einem der Ansprüche 10-11, umfassend:

Bestimmen einer Nachbarschaft für ein Gewebeelement aus der Vielzahl von Gewebeelementen, um die Gruppe von Gewebeelementen aus der Vielzahl von Gewebeelementen anzuzeigen, die nahe genug für eine Wechselwirkung mit dem Gewebeelement sind; und
Bestimmung einer Beschränkung für die Dichteverteilung für das Gewebeelement als Begrenzung für die kumulative Dichte für die Nachbarschaft, um dem Gewebe Inkompressibilität zu verleihen.

13. Verfahren nach einem der Ansprüche 10 bis 12, umfassend:

Bestimmen einer Nachbarschaft für ein Gewebeelement aus der Vielzahl von Gewebeelementen, um die Gruppe von Gewebeelementen aus der Vielzahl von Gewebeelementen anzuzeigen, die nahe genug für eine Wechselwirkung mit dem Gewebeelement sind; und
Bestimmung der Abstände zwischen den Gewebeelementen in der Nachbarschaft zur Ermittlung von Wiegekoeffizienten für die Interaktion zwischen den Gewebeelementen.

14. Verfahren nach einem der Ansprüche 9-13, bei dem das Modell verwendet wird, um eine haptische Rückmeldung zu erzeugen.

15. Computerprogrammprodukt, das Anweisungen enthält, die, wenn das Programm von einem Computer ausgeführt wird, den Computer veranlassen, das Verfahren nach einem der Ansprüche 9-14 auszuführen.

**Revendications**

1. Appareil (100) pour la préparation à une chirurgie, l'appareil (100) comprenant :

   un dispositif de suivi (110) pour représenter un instrument chirurgical (112) ;
   un dispositif d'affichage (120) pour afficher un sujet devant subir une chirurgie (130) ;
   un ou plusieurs processeurs (140) agencés pour recevoir des signaux correspondant au dispositif de suivi (110) pour déterminer la position d'un instrument chirurgical virtuel (112) suite au mouvement du dispositif de suivi (110) ; et
   une ou plusieurs mémoires (142) comprenant un code de programme informatique, les une ou plusieurs mémoires (142) et le code de programme informatique étant configurés pour amener les un ou plusieurs processeurs (140) à :

   générer un modèle d'une représentation tridimensionnelle d'un tissu correspondant au sujet devant subir une chirurgie (130), le modèle comprenant une pluralité d'éléments tissulaires agencés pour représenter ensemble le tissu ; sur la base de la position de l'instrument chirurgical virtuel (112) par rapport aux éléments tissulaires, modifier les une ou plusieurs contraintes pour indiquer qu'une coupe (132) a été réalisée sur le tissu (240) avec l'instrument chirurgical virtuel (112) ; et **caractérisé en ce que** les un ou plusieurs processeurs (140) sont en outre configurés pour
   déterminer un emplacement spatial pour chacun des éléments tissulaires avec un solveur local/global pour une équation d'état de mouvement pour les éléments tissulaires (230),
   lequel solveur local/global obtient de manière alternée un ensemble de solutions locales sous une ou plusieurs contraintes pour les éléments tissulaires indiquant une optimisation d'un potentiel élastique pour les éléments tissulaires et détermine les emplacements spatiaux qui optimisent l'équation d'état lorsque le potentiel élastique correspond aux solutions locales.

2. Appareil selon la revendication 1, dans lequel un emplacement spatial pour un élément tissulaire correspond à une distribution de densité et les une ou plusieurs contraintes comprennent une contrainte pour la distribution de densité.

3. Appareil selon la revendication 2, dans lequel l'ensemble de solutions locales est obtenu sous une contrainte indiquant qu'un gradient de déformation des éléments tissulaires pour indiquer l'élasticité du tissu correspond sensiblement à un gradient de déformation de référence.

4. Appareil selon la revendication 3, dans lequel l'obtention de l'ensemble de solutions locales comprend la détermination du gradient de déformation ou d'une indication de celui-ci, dans lequel la rotation est éliminée du gradient de déformation de référence ou de l'indication de celui-ci.

5. Appareil selon l'une quelconque des revendications 2 à 4, dans lequel les une ou plusieurs mémoires et le code de programme informatique sont configurés pour amener les un ou plusieurs processeurs à :

   déterminer un voisinage pour un élément tissulaire de la pluralité d'éléments tissulaires pour indiquer le groupe d'éléments tissulaires parmi la pluralité d'éléments tissulaires qui sont suffisamment proches pour interagir avec l'élément tissulaire ; et
   déterminer une contrainte pour la distribution de densité pour l'élément tissulaire en tant que limitation de densité cumulée pour le voisinage afin de fournir une incompressibilité au tissu.

6. Appareil selon la revendication 5, dans lequel la détermination de la contrainte pour la distribution de densité comprend la détermination d'un facteur d'échelle pour mettre à l'échelle les distances entre les éléments tissulaires dans le voisinage de sorte que la densité cumulée pour le voisinage soit limitée.

7. Appareil selon l'une quelconque des revendications 2 à 6, dans lequel les une ou plusieurs mémoires et le code de programme informatique sont configurés pour amener les un ou plusieurs processeurs à :

   déterminer un voisinage pour un élément tissulaire de la pluralité d'éléments tissulaires pour indiquer le groupe d'éléments tissulaires parmi la pluralité d'éléments tissulaires qui sont suffisamment proches pour interagir avec l'élément tissulaire ; et

déterminer les distances entre les éléments tissulaires du voisinage pour déterminer des coefficients de pondération pour l'interaction entre les éléments tissulaires.

8. Appareil selon l'une quelconque des revendications précédentes, dans lequel le dispositif de suivi comprend un actionneur pour une rétroaction haptique et l'appareil est agencé pour utiliser le modèle pour générer une rétroaction haptique vers l'actionneur.

9. Procédé comprenant :

la génération d'un modèle pour une représentation tridimensionnelle d'un tissu correspondant à un sujet devant subir une chirurgie (130),

le modèle comprenant une pluralité d'éléments tissulaires agencés pour représenter ensemble le tissu ; sur la base de la position de l'instrument chirurgical virtuel (112) par rapport aux éléments tissulaires, la modification des une ou plusieurs contraintes pour indiquer qu'une coupe (132) a été réalisée sur le tissu (240) avec l'instrument chirurgical virtuel (112) ; et **caractérisé par**

la détermination d'un emplacement spatial pour chacun des éléments tissulaires avec un solveur local/global pour une équation d'état de mouvement pour les éléments tissulaires (230),

lequel solveur local/global obtient de manière alternée un ensemble de solutions locales sous une ou plusieurs contraintes pour les éléments tissulaires indiquant une optimisation d'un potentiel élastique pour les éléments tissulaires et détermine les emplacements spatiaux qui optimisent l'équation d'état lorsque le potentiel élastique correspond aux solutions locales,

la réception d'un ou de plusieurs signaux pour déterminer la position d'un instrument chirurgical virtuel (112) suite au mouvement d'un dispositif de suivi (110).

10. Procédé selon la revendication 9, dans lequel un emplacement spatial correspond pour un élément tissulaire à une distribution de densité et les une ou plusieurs contraintes comprennent une contrainte pour la distribution de densité.

11. Procédé selon la revendication 10, dans lequel l'ensemble des solutions locales est obtenu sous une contrainte indiquant qu'un gradient de déformation des éléments tissulaires pour indiquer l'élasticité du tissu correspond sensiblement à un gradient de déformation de référence et dans lequel l'obtention de l'ensemble des solutions locales comprend la détermination du gradient de déformation ou d'une indication de celui-ci, dans lequel la rotation est éliminée du gradient de déformation de référence ou de l'indication de celui-ci.

12. Procédé selon l'une quelconque des revendications 10 et 11, comprenant :

la détermination d'un voisinage pour un élément tissulaire de la pluralité d'éléments tissulaires pour indiquer le groupe d'éléments tissulaires de la pluralité d'éléments tissulaires qui sont suffisamment proches pour une interaction avec l'élément tissulaire ; et

la détermination d'une contrainte pour la distribution de densité pour l'élément tissulaire en tant que limitation de densité cumulée du voisinage pour fournir une incompressibilité au tissu.

13. Procédé selon l'une quelconque des revendications 10 à 12, comprenant :

la détermination d'un voisinage pour un élément tissulaire de la pluralité d'éléments tissulaires pour indiquer le groupe d'éléments tissulaires de la pluralité d'éléments tissulaires qui sont suffisamment proches pour une interaction avec l'élément tissulaire ; et

la détermination des distances entre les éléments tissulaires dans le voisinage pour déterminer des coefficients de pondération pour l'interaction entre les éléments tissulaires.

14. Procédé selon l'une quelconque des revendications 9 à 13, comprenant l'utilisation du modèle pour provoquer la génération d'une rétroaction haptique.

15. Produit de programme informatique comprenant des instructions qui, lorsque le programme est exécuté par un ordinateur, amènent l'ordinateur à exécuter le procédé selon l'une quelconque des revendications 9 à 14.

EP 4 042 402 B1

FIG. 1

18

200

RECEIVE SIGNAL
FOR DETERMINING
POSITION OF VIRTUAL
SURGICAL INSTRUMENT — 210

GENERATE MODEL
FOR 3D REPRESENTATION
OF TISSUE — 220

DETERMINE SPATIAL LOCATION
FOR TISSUE ELEMENTS
WITH LOCAL/GLOBAL SOLVER
FOR STATE EQUATION — 230

ALTER CONSTRAINT
FOR LOCAL/GLOBAL SOLVER
TO INDICATE THAT
CUT HAS BEEN MADE TO TISSUE — 240

FIG. 2

FIG. 3

400

METHOD ∿ 200

REPRESENT
TISSUE ELEMENTS
WITH DENSITY DISTRIBUTIONS ∿ 310

DETERMINE NEIGHBORHOODS
FOR TISSUE ELEMENTS ∿ 420

LIMIT CUMULATIVE DENSITY
FOR NEIGHBORHOOD
FOR PROVIDING
INCOMPRESSIBILITY TO TISSUE ∿ 430

DETERMINE SCALING FACTOR
FOR DISTANCES
FOR LIMITING CUMULATIVE DENSITY
FOR NEIGHBORHOOD ∿ 440

USE DISTANCES BETWEEN
TISSUE ELEMENTS IN NEIGHBORHOOD
TO DETERMINE INTERACTION BETWEEN
SAID TISSUE ELEMENTS ∿ 450

FIG. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2010178644 A

**Non-patent literature cited in the description**

- **BOUAZIZ et al.** Projective Dynamics: Fusing Constraint Projections for Fast Simulation. *ACM Transactions on Graphics,* 2014, 33-4
- **MARTIN et al.** Example-based Elastic Materials. *ACM Transactions on Graphics,* 2011, 30-4

- **PEER et al.** An Implicit SPH Formulation for Incompressible Linearly Elastic Solids: Implicit Elastic SPH Solids. *Computer Graphics Forum,* 2017, 37-6